Europäisches Patentamt

(19) European Patent Office

Office européen des brevets

(11) Numéro de publication: **0 054 486**
**B1**

(12) **FASCICULE DE BREVET EUROPEEN**

(45) Date de publication du fascicule du brevet: **19.09.84**

(51) Int. Cl.³: **A 61 K 35/78**

(21) Numéro de dépôt: **81401975.8**

(22) Date de dépôt: **10.12.81**

(54) Composition pharmaceutique à usage topique à base d'un extrait total d'Hedysarum Fructescens Willd (lespedeza capitata michaux).

<table>
<tr><td>

(30) Priorité: **11.12.80 FR 8026342**

(43) Date de publication de la demande:
**23.06.82 Bulletin 82/25**

(45) Mention de la délivrance du brevet:
**19.09.84 Bulletin 84/38**

(84) Etats contractants désignés:
**AT BE CH DE GB IT LI LU NL SE**

(56) Documents cités:
**FR-A-1 201 726**
**FR-A-1 478 571**
**FR-E- 75 930**

**CHEMICAL ABSTRACTS, vol. 90, no. 19, 7 mai 1979, pages 300-301, réf. 148438c Columbus, Ohio, US LINARD A. et al.: "Flavonoids from Lespedeza capitata Michaux"**
**CHEMICAL ABSTRACTS, vol. 72, no. 26, 29 juin 1970, page 271, réf. 136325d Columbus, Ohio, US CALO, ALDO: "Separation, determination and evaluation of Lespedeza capitata flavones"**
**P.H. LIST "Hagers Handbuch der Pharmazeutischen Praxis", Vol. 5, Springer Verlag Berlin, 1976, p. 491**

</td><td>

(73) Titulaire: **Cervelle, Claude Marie Henri**
**27, rue Thiboumery**
**F-75015 Paris (FR)**

(72) Inventeur: **Cervelle, Claude Marie Henri**
**165, boulevard Haussmann**
**F-75008 Paris (FR)**
Inventeur: **Cervelle, Madeleine**
**165, boulevard Haussmann**
**F-75008 Paris (FR)**

(74) Mandataire: **Phélip, Bruno et al**
**c/o Cabinet Harlé & Phélip 21, rue de la Rochefoucauld**
**F-75009 Paris (FR)**

</td></tr>
</table>

Courier Press, Leamington Spa, England.

## Description

La présente invention concerne une composition pharmaceutique à usage topique à base d'un extrait total *d'Hedysarum Fructescens Willd* (Papilionacées), ladite composition étant utile notamment pour le traitement de maladies virales tégumentaires, en particulier pour le traitement de l'herpès, du zona et de la varicelle.

*Hedysarum Fructescens Willd* (Papilionacées), également dénommée *Lespedeza capitata* Michaux est une plante légumineuse à flavonoïdes. Elle est originaire d'Amérique du Nord. Sa culture a commencé en France dès 1950, dans le Val de Loire, à partir de graines provenant d'Amérique du Nord. Cette plante contient à titre de constituants essentiels des flavonoïdes, des tannins, des acides-phénols. Pour plus de détails sur cette plante et ses constituants on pourra se reporter à l'ouvrage de Alain G. CHARLES intitulé "Contribution à l'étude du *Lespedeza capitata* Michaux, légumineuse à flavonoïdes" éditions André Lesot (1962) Paris. L'étude chimique des flavonoïdes de cette plante a fait l'objet de la thèse présentée le 27 Octobre 1978, par Alain Linard pour l'obtention du titre de doctorat 3ème cycle dans des disciplines pharmaceutiques (Université René Descartes de Paris). A ce sujet on peut aussi citer l'article de M. TIN WA dans LLOYDIA, 32 (4) p. 509—11 (1969) qui concerne la séparation et l'identification de la Kampféritrine à partir de *Lespedeza capitata;* cette substance est un des flavonoïdes contenus dans Lespedeza capitata.

A titre de documents illustrant l'état antérieur de la technique, on peut encore citer les brevets français -1.478.571 et 1.201.726 (avec son addition n° 75.930).

Le brevet FR 1.478.571 concerne un procédé d'obtention à l'état sec ou en solution aqueuse des principes actifs hydrosolubles des végétaux de certaines classes. Il est indiqué dans la description de ce brevet que le principe du procédé consiste à éliminer, par des moyens physiques, les substances indésirables, telles que tannins, acides gras, pigments pour ne conserver que les substances pharmacologiquement actives, telles qu'alcaloïdes à l'état de sels ou flavonoïdes. Le *Lespedeza capitata* Michaux est cité à titre d'exemple de végétaux. Ainsi le brevet 1.478.571 concerne l'obtention d'un extrait duquel on élimine un certain nombre de substances. Il ne décrit pas un procédé pour obtenir un extrait total. D'ailleurs, les produits objet du brevet 1.478.571 sont des substances, telles que les alcaloïdes et les flavonoïdes, exemptes de tannins, d'acides gras et de pigments. Il ne s'agit pas d'un produit constitué d'une combinaison de flavonoïdes, de tannins et d'acides-phénols, et dépourvu d'alcaloïdes.

Le brevet 1.201.726 (et son addition n° 75 930) concernent l'obtention de dérivés flavoniques à partir de feuilles de certains végétaux. Ils ne concernent pas non plus un extrait total et un produit comprenant le combinaison mentionnée au paragraphe précédent.

La référence "Chemical Abstracts" vol. 90 n° 19, 7 Mai 1979, pages 300—301, n° 148438c est relative à un article de Linard et al qui concerne la composition de la *Lespedeza Capitata*. Il traite en particulier de certains flavonoïdes de cette plante, qui font l'objet d'une caractérisation particulière. La référence Chemical Abstracts vol. 72, n° 26 29 Juin 1970, page 271, n° 136 325d est relative à un article contribuant également à la connaissance chimique du végétal *Lespedeza Capitata* et en particulier de ses flavones.

Ces deux dernières références sont limitées à des indications sur les caractéristiques chimiques d'un végétal, le *Lespedeza Capitata*.

On siat que certains extraits de *Lespedeza Capitata* Michaux, les extraits flavonoïdiques, ont des propriétés pharmaceutiques et plus particulièrement des propriétés hypoazotémiques et hypocholestérolémiantes. A cet effet, on peut se référer notamment aux brevets spéciaux de médicaments (BSM) 296 M et 6058 M ainsi qu'au brevet BE 670.550.

Le brevet BSM 296 M concerne un lespédoside extrait de feuilles de *Lespedeza Capitata* ayant des propriétés hypoazotémiques et hypocholestérolémiantes. Ce lespédoside contient les dérivés flavoniques de la plante et est dépourvu des tannins. Il est administré par voie orale ou injectable.

Le brevet BSM 6058 M concerne un nouveau médicament dont le principe actif, dénommé Lespécapitoside, est un flavonoïde extrait des feuilles de *Lespedeza Capitata*. Ce lespécapitoside est obtenu par le procédé selon la brevet FR 1.500.157. Ce médicament possède des propriétés hypoazotémiques et antiathéromateuses, protégeant les parois artérielles contre les infiltrations lipidiques. Il est administrable par voie orale, intrapéritonéale ou intraveineuse.

Le brevet BE 670 550 concerne un médicament injectable obtenu à partir de *Lespedeza Capitata* et utilisable notamment pour le traitement de l'hyperazotémie. Ce médicament est une solution aqueuse injectable d'extrait lyophilisé de *Lespedeza Capitata* Michaux obtenu selon un procédé qui permet d'obtenir, à partir de certains végétaux, un extrait hydrosoluble débarrassé des tannins, des acides gras, des pigments, mais contenant, non altérées, les substances pharmacologiquement actives de la plante, en particulier les flavonoïdes et les alcaloïdes. Ainsi, l'extrait de *Lespedeza Capitata* mis en oeuvre dans ce médicament est dépourvu des tannins. De plus, le médicament n'est ni présenté, ni proposé en vue de l'administration topique.

Par ailleurs HARRY H. S. FONG et al [3. Pharm. Sci. *61* (11) p. 1818 (1972)] ont démontré que certaines plantes présentaient une activité antitumorale due aux tannins. Parmi ces plantes figure *Lespedeza Capitata* var. *velutina*. Les extraits de plantes utilisés lors de ces essais ont été obtenus à partir des plantes broyées par percolation avec un mélange 1:1 d'éthanol (95%) et d'eau, puis élimination de l'alcool par évaporation flash. Ces extraits ont été soumis à la section "Drug Research and Development" du National Cancer Institute pour déterminer leur activité antitumorale; cette détermination a été effectuée selon les protocoles décrits dans Cancer Chemotherapy reports n° 25, Décembre 1962; selon ces protocoles les produits à tester sont injectés par voie intrapéritonéale.

D'autre part, on a déjà utilisé des substances provenant des plantes du genre *Lespedeza* dans le traitement local des affections virales cutanées ou de la muqueuse buccale. On peut se référer à cet effet à la revue soviétique STOMATOLOGIJA S.S.S.R-1976- 55 N° 1 p. ·100—101 et à l'article de E. V. VERBENKO et al. dans Vestik Dermatol. i. Venereal., (1979) n° 6, p. 51—53. Ces articles sont relatifs au traitement par la Chélépine de certaines maladies cutanées ou buccales d'origine virale. Du point de vue chimique, la Chélépine est l'ensemble des flavonoïdes (kampférol, quercétine, orientine; homoorientine, lespédine, saponarétine et vitexine) isolés à partir de *Lespedeza hedysaroïdes* de la famille des légumineuses. Ces articles concernent donc l'utilisation, dans le traitement local de certaines maladies, d'un extrait exclusivement flavonique de *Lespedeza hedysaroïdes* qui est une espèce différente de *Lespedeza Capitata*. Des études chromatographiques ont en outre montré que les flavonoïdes de cet extrait étaient différents de ceux de *Lespedeza Capitata*.

On a maintenant trouvé que l'extrait total *de Lespedeza Capitata* Michaux ou *d'Hedysarum Fructescens Willd,* consistant essentiellement en une combinaison de flavonoïdes, de tannins catéchiques et d'acides phénols, dépourvue d'alcaloïdes présente d'excellentes propriétés pharmacologiques, appropriées pour le traitement des infections virales tégumentaires, en particulier de l'herpès, du zona et de la varicelle.

La présente invention concerne une composition pharmaceutique à usage topique, pour le traitement de maladies virales tégumentaires, caractérisée en ce qu'elle contient, à titre d'ingrédient actif, 2 à 10% en poids d'extrait total *d'Hedysarum Frustescens Willd* consistant essentiellement en une combinaison de flavonoïdes, de tannins catéchiques et d'acides phénols, ladite composition étant dépourvue d'alcaloïdes, en association avec un véhicule pharmaceutiquement acceptable, convenant pour l'administration topique.

L'extrait *d'Hedysarum Fructescens Willd* mis en oeuvre dans la composition pharmaceutique selon l'invention est de couleur jaune-brun; cette couleur est variable selon le mode d'extraction. Il est partiellement soluble, en particulier dans l'eau et l'éthanol.

L'extrait total *d'Hedysarum Frustescens Willd*

peut être obtenu par extraction à l'alcool éthylique à partir de la plante sèche, de la plante fraîche, éventuellement débarrassée au préalable des graisses, des cires et de la chlorophylle ou par extraction à l'alcool éthylique après macération de la plante fraîche dans du propylène-glycol.

On décrira ci-après, à titre non limitatif différents modes d'extraction de l'extrait total *d'Hedysarum Fructescens Willd*. Ces différents modes d'obtention donnent des résultats comparables quant à la composition qualitative; cependant, la richesse relative en principes actifs (flavonoïdes, tannins catéchiques et acides-phénols) varie avec le mode d'obtention.

Selon un mode d'obtention, on épuise la plante sèche (parties aériennes) par lixiviation alcoolique par exemple avec l'alcool éthylique à 60% en volume. Le volume de l'extrait hydro-alcoolique est ensuite réduit par concentration sous vide. La solution est laissée décanter, puis est ensuite filtrée. La concentration sous vide est réalisée jusqu'à consistance molle du résidu. Après déshydratation en étude sous vide, l'extrait est pulvérisé et tamisé; on obtient ainsi un extrait sec classique.

Un autre mode d'obtention consiste à épuiser la plante fraîche (parties aériennes) par lixiviation alcoolique par exemple avec de l'alcool éthylique à 95% en volume. Le volume de l'extrait hydro-alcoolique est ensuite réduit par concentration sous vide. On ajoute alors de la poudre inerte, par exemple de la lévilite, à environ 25% par rapport à l'extrait sec calculé dans l'étape précédente. Après homogénéisation, le mélange est réduit en poudre par nébulisation. On obtient ainsi un extrait total se présentant sous la forme d'un nébulisat.

Selon un autre mode d'obtention, on peut au préalable éliminer de la plante les graisses, les cires et la chlorophylle par traitement à l'aide de différents solvants organiques (acétone, éther de pétrole, chloroforme et produits similaires) et ensuite épuiser le produit résultant à l'aide d'alcool éthylique selon le mode opératoire ci-dessus.

Selon un autre mode opératoire, la plante aérienne fraîche est broyée et traitée par macération dans la propylène-glycol ou un autre glycol, pendant environ 48 heures et est ensuite soumise à une lixiviation à l'alcool et à une filtration. On obtient ainsi un soluté glyco-alcoolique.

La caractérissation des différents principes actifs de l'extrait total *d'Hedysarum Fructescens Willd* mis en oeuvre dans la composition selon l'invention, à savoir les flavonoïdes, les tannins et les acides phénols, peut être effectuée par chromatographie bidimensionnelle en couche mince de cellulose.

La caractérisation des tannins catéchiques est réalisée selon la technique définie par HASLAM E. [Chemistry of Vegetable Tannins, Academic Press, London and New-York (1966)], par chromatographie bidimensionnelle en couche mince de cellulose. L'élution est effectuée avec les systèmes ci-après:

1) butanol secondaire/acide acétique/eau dans un rapport volumique de 14-1-5 et
2) acide acétique à 6% dans l'eau.

La révélation est effectuée à l'aide d'acide paratoluène-sulfonique à 5% dans l'alcool éthylique et par chauffage à 100°C pendant 2,5 minutes. La présence de tannins catéchiques est caractérisée par une coloration brune.

La caractérisation des acides-phénols est aussi réalisée par chromatographie bidimensionnelle en couche mince de cellulose, selon la technique définie par PARIS (R. R.) et MURGU (L.) ["Sur les composés polyphénoliques de SPIRAEA CRENATA. L. "Plantes Médicinales et Phytothérapie, *4*, n° 2, p. 138—149, (1970)]. Les systèmes d'élusion sont les suivants:

1) butanol/acide acétique/eau dans un rapport volumique de 4-1-5.
2) acide acétique/eau (rapport volumique 15/85). La révélation est réalisée à l'aide de chlorure ferrique à 5% dans l'eau ou à l'aide d'un rayonnement ultraviolet.

La présence d'acides phénols est décelée par

— une fluorescence bleue (en U.V. à 350 nm) ou brun-mat (en U.V. à 254 nm);
— une coloration bleue à bleu-vert avec le chlorure ferrique;
— une révélation à la para-nitraniline diazotée suivie d'une seconde pulvérisation d'une solution de carbonate disodique à 2%; les colorations obtenues sont spécifiques des acides phénols et elles sont comparées avec celles d'acides-phénols de référence.

La mise en évidence qualitative des flavonoïdes est effectuée par chromatographie bidimensionnelle sur couche mince de cellulose, selon la méthode de HARBORNE et WILLIAMS [Flavone and Flavonol glycosides in Harborne (J. B.), MABRY (T. J.) and MABRY (H). The flavonoids Chapman and Hall London 376—441 (1975)]. Les solvants d'élution sont les suivants:

1) butanol/acide acétique/eau dans le rapport volumique 4-1-5,
2) acide acétique/eau dans un rapport volumique 15—85.

La révélation est effectuée avec du trichlorore d'aluminium à 2% dans l'alcool à 96% ou avec de l'acétate basique de plomb (solution officinale). L'observation est réalisée en lumière ultraviolette avant et après révélation des chromatogrammes. Lorsque le révélateur est le trichlorure d'aluminium, les flavonoïdes présentent une fluorescence jaune plus ou moins intense, tandis que lorsque le révélateur est de l'acétate basique de plomb, les orthodiphénols présentant une fluorescence orangée en lumière ultraviolette, alors que pour les autres phénols on observe une fluorescence jaune ou jaune-vert. Pour plus de détail au sujet de la caractérisation des flavonoïdes on pourra se référer à la thèse de Alain LINARD citée précédemment.

L'analyse quantitative des trois principes actifs de l'extrait total *d'Hedysarum Fructescens Willd* est réalisée selon les techniques ci-après.

1. Dosage des flavonoïdes
Ce dosage est réalisé selon la méthode décrite par HORHAMMER L. et HANSEL R. 1951 [Nachweiss und Bestimmung von Rutin neben Quercetin Arch. Pharm. *284* n° 56 p. 276—280]. Cette méthode sera rappelée ci-après:

a) dans le cas des extraits pulvérulents on prépare une solution d'essai ou solution à doser de la façon suivante:

Dans un bécher de 150 ml, on place une prise d'essai de poudre exactement pesée, voisine de 1,50 g. On ajoute 60 à 70 ml d'alcool éthylique à 70° bouillant. On maintient le mélange sous agitation magnétique chauffante pendant 30 minutes. On filtre sur verre fritté et on recueille le filtrat quantitativement dans une fiole jaugée de 100 ml. On rince le verre fritté avec de l'alcool à 70° bouillant. On ajoute cette fraction au filtrat. Après refroidissement, on complète au volume de 100 ml avec de l'alcool éthylique à 70°. Le dosage est réalisé directement à partir de l'extrait hydro-alcoolique obtenu.

b) dans le cas d'un extrait liquide, le dosage est réalisé sur l'extrait lui-même, dilué au quart.

Les réactifs utilisés dans ce dosage sont les suivants:

a) solution d'oxychlorure de zirconium à 3% dans l'eau;
b) solution tampon obtenue avec le mélange suivant:

| | |
|---|---|
| acide acétique glacial | 24 ml |
| acétate de sodium cristallisé | 27 g |
| eau | 200 ml |
| alcool éthylique à 60° | 500 ml |

Le dosage des flavonoïdes est effectué de la façon suivante:
A 0,1 ml de la solution à doser on ajoute 5 ml de solution tampon et 1 ml de solution aqueuse d'oxychlorure de zirconium à 3% (P/V). Après 5 minutes, la densité optique est mesurée à 400 nm par rapport à une solution témoin dans laquelle la solution à doser est remplacée par de l'alcool éthylique à 60°.

La teneur en flavonoïdes est obtenue en se reportant à une courbe d'étalonnage établie pour une concentration en rutine de 0 à 200 μg (résultats en g pour 100 g d'extrait ou g pour 100 ml dans le cas d'extrait liquide).

2. Dosage des tannins catéchniques

Le dosage des tannins catéchniques (y compris les catéchines) a été effectué à l'aide du *réactif de Stiasny* (2 volumes de formol à 30% pour 1 volume d'acide chlorhydrique concentré). [Selon la méthode décrite par A. CHARLES dans l'ouvrage "Contribution à l'Etude de *Lespedeza capitata* Michaux" cité précédemment].

Selon cette méthode on laisse 2 grammes d'extrait pulvérulent en contact pendant 2 heures avec 180 ml d'eau à 80°C. On filtre sur verre fritté, on lave le résidu à l'eau chaude jusqu'à obtention d'un volume final de 200 ml. Au filtrat, on ajoute 100 ml de réactif de Stiasny. Après repos de 24 heures, le précipité est recueilli sur un filtre fritté taré, lavé jusqu'à neutralité et desséché à 100°C à poids constant.

On obtient ainsi la proportion de tannins catéchiques (et de catéchines) en g pour 100 g d'extrait pulvérulent.

Dans le cas d'un extrait liquide, à 100 ml d'extrait, on ajoute 50 ml de réactif de Stiasny. Ensuite le processus est identique à celui de l'extrait sec ci-dessus.

3. Dosages des acides-phénols

a) extrait pulvérulent:

Un infusé à 10% (P/V), fait extemporanément est acidifié par l'acide sulfurique à 10% (V/V). Après refroidissement on extrait par l'éther éthylique. La solution éthérée est épuisée par une solution de bicarbonate de sodium à 2% (éther I). Après acidification de la phase aqueuse par l'acide sulfurique à 10%, elle est épuisée par l'éther éthylique (éther II). Les fractions éthérées I et II sont réunies, séchées sur sulfate de sodium anhydre, évaporées à sec sous pression réduite. Elles sont conservées, sous vide, en présence d'anhydride phosphorique et pesées à poids constant.

On obtient un résultat exprimé en g pour 100 g d'extrait pulvérulent.

b) extrait liquide:

Si l'extrait contient de l'alcool éthylique, 100 ml de l'extrait sont évaporés sous pression réduite. Le volume de l'extrait est ajusté à 100 ml avec de l'eau, puis acidifié par l'acide sulfurique à 10%. Ensuite le processus est identique à celui de l'extrait pulvérulent ci-dessus. La teneur en acides phénols est exprimée en g pour 100 ml d'extrait.

L'activité thérapeutique remarquable de l'extrait total *d'Hedysarum Fructescens Wills* résulte de la synergie entre les trois principes actifs contenus dans cet extrait: les falvonoïdes, les tannins catéchiques et les acides phénols.

L'extrait total *d'Hedysarum Fructescens Willd* possède des propriétés curatives très intéressantes pour traiter l'herpès, le zona et également d'autres manifestations virales de la peau et des muqueuses. Contrairement à certains produits disponibles sur le marché et utilisés dans ce type de traitement, l'extrait total *d'Hedysarum Fructescens Willd* ne présente aucune toxicité et on n'a noté aucune contre-indication.

L'extrait total *d'Hedysarum Fructescens Willd* agite efficacement même si le traitement n'est entrepris que lorsque la lésion a dépassé le premier stade d'évolution.

L'extrait total *d'Hedysarum Fructescens Willd* peut être appliqué par voie topique sous différentes formes en combinaison avec un véhicule pharmaceutiquement acceptable.

Ainsi, la composition selon l'invention se présente sous la forme de solutions, par exemple de solutions aqueuses ou de solutions hydro-alcooliques contenant 2 à 10% en poids de l'extrait total.

La composition, selon l'invention, peut également se présenter sous la forme de crème dermique, le véhicule pharmaceutiquement acceptable étant alors un excipient pénétrant tel qu'un mélange équilibré de polyglycols. Ces crèmes contiennent généralement 2 à 10% en poids de l'extrait total *d'Hedysarum Fructescens Willd.* La composition selon l'invention peut également être administrée sous la forme d'un collyre pour le traitement des lésions oculaires, telles que l'herpès cornéen ou le zona ophtalmique.

Le véhicule pharmaceutique acceptable, qui convient deux fins de l'invention, est donc un véhicule qui permet une application topique et peut être notamment l'eau, les mélanges eau-alcool, les solutions physiologiques, les crèmes, onguents et autres véhicules solides, pâteux ou liquides utilisés de manière classique. A titre de base de crème ou d'onguent on utilise toute substance ou combinaison de substances utilisées à cet effet, telles que des polyglycols.

L'invention va être maintenant décrite plus en détail dans les exemples illustratifs et non limitatifs ci-après:

Exemple 1
Préparation de l'extrait total *d'Hedysarum Fructescens Willd*

A. extrait sec classique

On a épuisé par lixiviation à l'alcool éthylique à 60°, 100 kg de plantes sèches provenant de BEAULIEU (Loiret) France en utilisant 1000 litres d'alcool éthylique. On a réduit par concentration sous vide la solution hydro-alcoolique obtenue. On l'a laissée décanter, puis on l'a filtrée. Après déshydratation en étuve sous vide, l'extrait a été pulvérisé et tamisé. On a obtenu environ 10 kg d'extrait. La caractérisation des flavonoïdes, des acides phénols et des tannins réalisée selon les modes opératoires définis précédemment a donné un résultat positif; par contre, on n'a pas révélé la présence d'alcaloïdes.

L'analyse quantitative de l'extrait ainsi obtenu, réalisée selon les modes opératoires définis ci-dessus, était sensiblement la suivante (en poids par poids d'extrait):

| | |
|---|---|
| flavonoïdes | 9 à 12% |
| tannins | 32 à 35% |
| aices-phénols | 0,4 à 0,5% |

B. nébulisat

L'équivalent de 200 kg de plantes sèches, traité par de l'alcool éthylique à 95°, a donné 1000 litres d'extrait hydro-alcoolique. Cet extrait hydro-alcoolique a été réduit par concentration sous vide. On a ensuite ajouté 25% de poudre inerte (lévilite) par rapport à l'extrait sec. Après homogénéisation, le mélange a été réduit en poudre par nébulisation. On a alors obtenu 8 kg d'extrait à 25% de poudre inerte.

La caractérisation des principes actifs de cet extrait (flavonoïdes, acides phénols et tannins) effectuée selon les modes opératoires ci-dessus a donné un résultat positif. On n'a pas décelé d'alcaloïdes dans l'extrait ainsi obtenu.

L'analyse quantitative de l'extrait ainsi obtenue était la suivante (en poids par poids d'extrait):

| | |
|---|---|
| flavonoïdes | 9 à 12% |
| acides-phénols | 0,6 à 0,9% |
| tannins | 38 à 40% |

C. soluté glyco-alcoolique

On a fait macérer pendant 48 heures 100 kg de plantes fraîches provenant également de BEAULIEU (Loiret) France dans 25 kg de propylène-glycol. On a ensuite effectué une lixiviation avec 75 kg d'alcool éthylique à 30%. Après décantation, on a filtré et on a obtenu 100 kg d'extrait.

La caractérisation des principes actifs de cet extrait (flavonoïdes, acides-phénols et tannins) effectuée selon les modes opératoires ci-dessus a donné un résultat positif. On n'a pas décelé d'alcaloïdes dans l'extrait ainsi obtenu.

L'analyse quantitative de l'extrait ainsi obtenu était la suivante (en poids par volume d'extrait):

| | |
|---|---|
| flavonoïdes | 0,3 à 0,4% |
| acides-phénols | 0,03 à 0,5% |
| tannins | 1,0 à 1,5% |

Exemple 2
Essais cliniques

On a utilisé une solution hydro-alcoolique à 5% de l'extrait total obtenu selon l'exemple 1((A) ou une crème dermique au même dosage, le véhicule étant constitué d'un mélange équilibré de poly-glycols. La crème, évitant les coulées de liquide, est parfois plus pratique à utiliser. L'activité de l'une ou l'autre forme était sensiblement la même.

L'application a été réalisée soit par tamponnage avec la solution aqueuse, soit par application avec léger message avec la crème dermique. Le traitement a été réalisé dans chaque cas pendant 2 ou 3 jours, ou plus si nécessaire, à raison de 2 à 4 fois par jour.

ler cas: herpès lombaire récidivant

Un homme de 50 ans avait un herpès lombaire récidivant (tous les deux mois en moyenne, surtout l'hiver). Les poussées d'herpès semblaient être provoquées par des phénomènes digestifs ou être une conséquence de la diminution de la résistance immunologique du sujet (par exemple fatigue ou préoccupation, coryza, etc ... ).

Les poussées étaient traitées par les médications habituelles, qui n'amenaient qu'un léger raccourcissement de l'évolution des vésicules. La vaccination herpétique avait été sans effet notable.

On a tamponné 2 à 3 fois par jour la lésion avec la solution hydro-alcoolique selon l'invention. Les résultats obtenus sont les suivants:

En Septembre 1978:

Application tardive de la solution selon l'invention. On a obtenu le dessèchement des vésicules en 24 heures, puis apparition de croûte et la chute de celle-ci en trois jours. La disparition de la sensation de brûlure a eu lieu dès la première application le matin, celle-ci réapparaissant le soir et redisparaissant dès la seconde application, pour ne plus revenir.

La réaction ganglionnaire et la lourdeur de la jambe se sont atténuées en 24 heures.

Novembre 1978:

Applications de la composition selon l'invention dès la phase pré-vésiculaire. On a noté la disparition totale de la sensation de brûlure dès la première application, et la disparition définitive de la tension oedémateuse de la peau à la 3ème application.

Février 1979:

Même traitement précoce, même résultat.

En mai et octobre 1979 ainsi qu'en mars 1980 on a procédé au même traitement précoce et on a obtenu le même résultat.

Aucune poussée herpétique n'a été notée depuis mars 1980.

2ème cas: Herpès récidivant de la commissure labiale

Une femme de 58 ans présentant un herpès récidivant de la commissure labiale a été traitée à 2 reprises (en octobre 1979, et mars 1980), mais toujours tardivement car la malade ne s'est préoccupée de traiter son herpès qu'après la formation de vésicules. Les poussées antérieures duraient une quinzaine de jours à chaque fois.

Avec la composition selon l'invention on a obtenu le dessèchement des vésicules en 24 heures par application 3 fois par jours après les repas. La croûte est tombée 3 jours après.

La sensation de brûlure a disparu dès les premières applications.

3ème cas: Herpès cataménial récidivant

Une femme de 41 ans présentait un herpès cataménial récidivant. Le traitement avec la composition selon l'invention a été réalisé dès le début des crises (octobre 1979 et mars 1980) par la crème dermique ou par la solution aqueuse à 5%.

Alors que, dans les crises antérieures, et malgré des applications locales de divers produits, la poussée plus ou moins légèrement raccourcie restait très gênante pour le sujet, on a constaté lors des deux traitements avec la composition selon l'invention ou avortement de la lésion en moins de 48 heures avec disparition de la gêne locale.

Une poussée en décembre 1979, traitée après que les vésicules se sont formées avec la composition de l'invention a été stoppée et la lésion cicatrisée en 48 heures.

Utilisée précocement dans l'herpès dermique ou muqueux, la composition selon l'invention (solution aqueuse hydro-alcoolique ou crème) a fait avorter la lésion sand qu'il y ait formation de vésicules; la sensation de brûlure ou démangeaison a disparu presque instantanément. En 24 heures la peau ou la muqueuse a retrouvé son intégrité, la réaction ganglionnaire éventuelle avait disparu. Il convient de poursuivre cependant les applications pendant 2 ou 3 jours.

Si la lésion en était déjà au stade vésiculaire, l'application de la composition selon l'invention a accéléré l'évolution: au lieu de 10 à 15 jours, le dessèchement des vésicules et la cicatrisation n'ont demandé que 2 à 3 jours.

La sensation de brûlure ou démangeaison a disparu presque instantanément, les réactions ganglionnaires se sont effacées rapidement.

Dans le traitement de l'herpès récidivant, il convient de répéter la cure dès l'apparition d'une nouvelle poussée herpétique. Après quelques traitements, on a constaté un espacement, puis une disparition des poussées herpétiques.

La composition selon l'invention à 5% d'extrait total (sous forme de solution aqueuse hydro-alcoolique ou de crème dermique) a aussi été utilisée pour le traitement de zonas. Dans chaque cas on a noté une amélioration sensible au bout d'une semaine de traitement dans la forme bulbeuse, au bout d'un temps plus long pour les autres formes. Les douleurs ont été notablement atténuées.

L'invention procure donc une composition pharmaceutique non toxique, dénuée d'effets secondaires, et applicable par voie locale pour le traitement des maladies virales tégumentaires les plus variées.

**Revendications pour les Etats Contractants: BE, CH, DE, GB, IT, LI, LU, NL, SE**

1. Composition pharmaceutique à usage topique, pour le traitement de maladies virales tégumentaires, caractérisée en ce qu'elle contient, à tire d'ingrédient actif, 2 à 10% en poids d'extrait total *d'Hedysarum Fructescens Willd (Lespedeza capitata Michaux)* consistant essentiellement en une combinaison de flavonoïdes, de tannins catéchiques et d'acides-phénols, dépourvue d'alcaloïdes, en association avec un véhicule pharmaceutiquement acceptable, convenant pour l'administration topique.

2. Composition selon la revendication 1, caractérisée en ce que le véhicule pharmaceutiquement acceptable est de l'eau, un mélange eau-alcool ou une solution physiologique.

3. Composition selon la revendication 1, caractérisée en ce que le véhicule pharmaceutiquement acceptable est un onguent à base de polyglycols.

4. Composition selon l'une quelconque des revendications 1 à 3, caractérisée en ce que l'extrait *d'Hedysarum Fructescens Willd (Lespedeza capitata Michaux)* est obtenu par lixiviation de la plante sèche (partie aérienne) à l'alcool, tel que l'alcool éthylique, réduction par concentration sous vide de l'extrait hydro-alcoolique ainsi obtenu, pulvérisation et tamisage, ledit extrait se présentant sous la forme d'un extrait sec.

5. Composition selon la revendication 4, caractérisée en ce que la plante est au préalable débarrassée des graisses, des cires et de la chlorophylle.

6. Composition selon l'une quelconque des revendications 1 à 5, caractérisée en ce que l'extrait *d'Hedysarum Fructescens Willd (Lespedeza capitata Michaux)* est obtenu par lixiviation de la plante fraîche à l'alcool, par exemple l'alcool éthylique, réduction par concentration sous vide de l'extrait hydro-alcoolique, addition de poudre inerte à raison de 25% en poids par rapport à l'extrait hydro-alcoolique, homogénéisation et réduction en poudre par nébulisation.

7. Composition selon l'une quelconque des revendications 1 à 5, caractérisée en ce que l'extrait *d'Hedysarum Fructescens Willd (Lespedeza capitata Michaux)* est obtenu par traitement de la plante fraîche par macération dans un glycol pendant environ 48 heures, puis lixiviation à l'alcool et filtration, l'extrait ainsi obtenu se présentant sous la forme d'un soluté glycoalcoolique.

8. Composition selon l'une quelconque des revendications 1 à 7, pour le traitement de l'herpès, du zona et de la varicelle, caractérisée en ce qu'elle contient 2 à 10% en poids et spécialement 5% en poids de l'extrait *d'Hedysarum Fructescens Willd (Lespedeza capitata Michaux)*.

**Revendications pour l'Etat Contractant: AT**

1. Procédé de préparation d'une composition pharmaceutique à usage topique pour le traitement de maladies virales tégumentaires, caractérisé en ce qu'on mélange 2 à 10% en poids d'extrait total *d'Hedysarum Fructescens Willd* (Lespedeza capitata Michaux) obtenu par lixiviation de la plante à l'alcool, tel que l'alcool éthylique, réduction par concentration sous vide de l'extrait hydro-alcoolique ainsi obtenu, pulvérisation et tamisage, avec un véhicule pharmaceutiquement acceptable convenant pour l'administration topique.

2. Procédé selon la revendication 1, caractérisé en ce que la plante est au préalable débarrassée des graisses, des cires et de la chlorophylle.

3. Procédé selon l'une des revendications 1 ou 2 caractérisé en ce que la lixiviation de la plante est faite après macération de la plante fraîche dans un glycol pendant environ 48 heures, ce qui donne après lixiviation un extrait sous forme de soluté glyco-alcoolique.

4. Procédé selon l'une des revendications caractérisé en ce que après réduction par concentration sous vide de l'extrait hydro-alcoolique obtenu par lixiviation de la plante fraîche, on ajoute une poudre inerte à raison de 25% en poids par rapport à l'extrait, on homogénéise et réduit en poudre par nébulisation.

**Patentansprüche für die Vertragsstaaten: BE, CH, LI, DE, GB, IT, LU, NL, SE**

1. Pharmazeutische Zubereitung für die örtliche Anwendung zur Behandlung von viralen Hautkrankheiten, dadurch gekennzeichnet, daß sie als Wirkstoff 2 bis 10 Gew.-% Totalextrakt von Hedysarum Fructescens Willd (Lespedeza capitata Michaux), der im wesentlichen aus einer von Alkaloiden befreiten Kombination von Flavonoiden, von Catechintanninen und Phenolsäuren besteht, in Verbindung mit einem pharmazeutisch annehmbaren, für die örtliche Anwendung geeigneten Träger enthält.

2. Zubereitung nach Anspruch 1, dadurch gekennzeichnet, daß der pharmazeutische annehmbare Träger Wasser, ein Wasser-Alkohol-Gemisch oder eine physiologische Lösung ist.

3. Zubereitung nach Anspruch 1, dadurch gekennzeichnet, daß der pharmazeutisch annehmbare Träger eine Salbe auf Basis von Polyglykolen ist.

4. Zubereitung nach irgendeinem der Ansprüche 1 bis 3, dadurch gekennzeichnet, daß der Extrakt von Hedysarum Fructescens Willd (Lespedeza capitata Michaux) durch Auslaugen der trockenen Pflanze (Luftteil) mit Alkohol, z.B. Ethylalkohol, Reduktion des so erhaltenen Wasser-Alkohol-Extrakts durch Konzentrieren unter Vakuum, Pulverisierung und Sieben erhalten wird, wobei der Extrakt in Form eines trockenen Extrakts vorliegt.

5. Zubereitung nach Anspruch 4, dadurch gekennzeichnet, daß die Pflanze vorher von Fetten, Wachsen und Chlorophyll befreit wird.

6. Zubereitung nach irgendeinem der Ansprüche 1 bis 5, dadurch gekennzeichnet, daß der Extrakt von Hedysarum Fructescens Willd (Lespedeza capitata Michaux) durch Auslaugen der frischen Pflanze mit Alkohol, z.B. Ethylalkohol, Reduktion des Wasser-Alkohol-Extrakts durch Konzentrieren unter Vakuum, Zugabe von inertem Pulver in einer Menge von 25 Gew.-%, bezogen auf den Wasser-Alkohol-Extrakt, Homogenisierung und Zerkleinierung zu Pulver durch Zerstäubung erhalten ist.

7. Zubereitung nach irgendeinem der Ansprüche 1 bis 5, dadurch gekennzeichnet, daß der Extrakt von Hedysarum Fructescens Willd (Lespedeza capitata Michaux) durch Behandlung der frischen Pflanze durch Mazeration während etwa 48 Stunden in einem Glykol, anschließendes Auslaugen mit Alkohol und Filtration erhalten wird und der so erhaltene Extrakt in Form einer glykoalkoholischen Lösung vorliegt.

8. Zubereitung nach irgendeinem der Ansprüche 1 bis 7 für die Behandlung von Herpes, Gürtelrose und Varicellen, dadurch gekennzeichnet, daß sie 2 bis 10 Gew.-%, insbesondere 5 Gew.-% Extrakt von Hedysarum Fructescens Willd (Lespedeza capitata Michaux) enthält.

**Patentansprüche für der Vertragsstaat: AT**

1. Verfahren zur Herstellung einer pharmazeutischen Zubereitung für die Behandlung von viralen Hautkrankheiten, dadurch gekennzeichnet, daß man 2 bis 10 Gew.-% Totalextrakt von Hedysarum Fructescens Willd (Lespedeza capitata Michaux), der durch Auslaugen der Pflanze mit Alkohol, z.B. Ethylalkohol, Zerkleinerung des so erhaltenen Wasser-Alkohol-Extrakts durch Konzentieren unter Vakuum, Zerstäuben und Sieben erhalten worden ist, mit einem für die örtliche Anwendung geeigneten pharmazeutisch annehmbaren Träger mischt.

2. Verfahren nach Anspruch 1, dadurch gekennzeichnet, daß die Pflanze vorher von Fetten, Wachsen und von Chlorophyll befreit worden ist.

3. Verfahren nach einem der Ansprüche 1 oder 2, dadurch gekennzeichnet, daß das Auslaugen der Pflanze nach Mazeration der frischen Pflanze in einem Glykol während etwa 48 Stunden erfolgt, was nach Auslaugung einen Extrakt in Form einer glykoalkoholischen Lösung ergibt.

4. Verfahren nach einem der vorliegenden Ansprüche, dadurch gekennzeichnet, daß man nach der Zerkleinerung des durch Auslaugen der frischen Pflanze erhaltenen Wasser-Alkohol-Extrakts durch Konzentrieren unter Vakuum ein inertes Pulver in einer Menge von 25 Gew.-%, bezogen auf den Extrakt, zugibt, homogenisiert und durch Zerstäubung zu Pulver zerkleinert.

**Claims for the Contracting States: BE, CH, LI, DE, GB, IT, LU, NL, SE**

1. Pharmaceutical composition for topical use, for the treatment of tegumentary viral disorders, characterised in that it comprises as the effective ingredient, from 2 to 10% by weight of total extract of *Hedysarum Fructescens Willd (Lespedeza capitata Michaux)* consisting essentially of a combination of flavonoids, catechol tannins and phenolic acids, devoid of alkaloids, together with a pharmaceutically acceptable vehicle suitable for topical administration.

2. Composition according to Claim 1, characterised in that the pharmaceutically acceptable vehicle is water, a water-alcohol mixture or a physiological solution.

3. Composition according to Claim 1, characterised in that the pharmaceutically acceptable vehicle is an unguent comprising polyglycols.

4. Composition according to any one of Claims 1 to 3, characterised in that the extract of *Hedysarum Fructescens Willd (Lespedeza capitata Michaux)* is obtained by lixiviating the dry plant (above-ground portion) with an alcohol, such as ethyl alcohol, concentrating the aqueous alcoholic extract thus obtained in vacuo, spray-drying and sieving, the said extract being in the form of a dry extract.

5. Composition according to Claim 4, characterised in that the plant is beforehand freed from fats, waxes and chlorophyll.

6. Composition according to any one of Claims 1 to 5, characterised in that the extract of *Hedysarum Fructescens Willd (Lespedeza capitata Michaux)* is obtained by lixiviating the fresh plant with an alcohol, for example ethyl alcohol, concentrating the aqueous alcoholic extract in vacuo, adding an inert powder in an amount of 25% by weight of the aqueous alcoholic extract, homogenising the mixture and reducing it to a powder, by atomising.

7. Composition according to any one of Claims 1 to 5, characterised in that the extract of *Hedysarum Fructescens Willd (Lespedeza capitata Michaux)* is obtained by treating the fresh plant by maceration in a glycol for about 48 hours followed by lixiviation with an alcohol and filtration, the extract thus obtained being in the form of a glycol/alcohol solution.

8. Composition according to any one of Claims 1 to 7, for the treatment of herpes, zona and varicella, characterised in that it contains from 2 to 10% by weight and particularly 5% by weight of the extract of *Hedysarum Fructescens Willd (Lespedeza capitata Michaux)*.

**Claims for the Contracting State: AT**

1. Process for the preparation of a pharmaceutical composition for topical use, for the treatment of tegumentary viral disorders, characterised in that from 2 to 10% by weight of total extract of *Hedysarum Fructescens Willd* (Lespedeza capitata Michaux), obtained by lixiviating the plant with an alcohol, such as ethyl alcohol, concentrating the aqueous alcoholic extract thus obtained in vacuo, spraying and sieving, are mixed with a pharmaceutically acceptable vehicle suitable for topical administration.

2. Process according to Claim 1, characterised in that the plant is beforehand freed from fats, waxes and chlorophyll.

3. Process according to either of Claims 1 and 2, characterised in that the lixiviation of the plant is carried out after macerating the fresh plant in a glycol for about 48 hours, thereby obtaining, after lixiviation, an extract in the form of a glycol/alcohol solution.

4. Process according to any one of preceding claims characterised in that after the aqueous alcoholic extract obtained by lixiviation of the fresh plant has been concentrated in vacuo, an inert powder is added in an amount of 25% by weight of the extract, and the mixture is homogenised and reduced to a powder by atomising.